# EUROPEAN PATENT SPECIFICATION

(11) **EP 1 227 795 B1**
(45) Date of publication and mention of the grant of the patent: **11.02.2004**
(21) Application number: 00979540.2
(22) Date of filing: 30.10.2000
(51) Int. Cl.: A61K 9/127, A61P 35/00

(54) **LIPID COMPLEX OF ALKYCYCLINES**
LIPIDKOMPLEXE VON ALKYLCYCLINEN
COMPLEXE LIPIDIQUE D'ALKYCYCLINES

(30) Priority: 12.11.1999 GB 9926843
(43) Date of publication of application: 07.08.2002
(73) Proprietor: Pharmacia Italia S.p.A., 20152 Milano (IT)
(72) Inventor: CHERIAN, Mathew, Elmhurst, Illinois 60126, USA (US); BIANCHI CARNEVALE, Claudia, I-27100 Gropello Cairoli (IT); COLAJORI, Elena, I-20010 Boffalora Ticino (IT); VALOTA, Olga, I-20025 Legnano (IT)
(86) International application number: PCT/EP2000/010997
(87) International publication number: WO 2001/035937

(56) References cited:
- EP-A- 0 800 822
- WO-A-99/48503

## Description

The present invention relates to a buffered lyophilized form of a lipid complex of an alkycycline of formula Ia 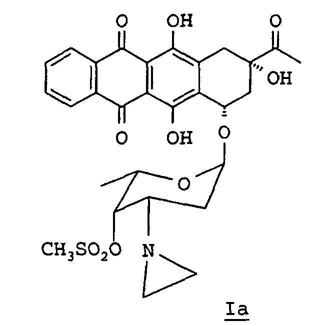 that can be reconstituted and administered to a patient in need of anti-tumor treatment. The chemical name of the alkycycline of the formula Ia is 4-demethoxy-3'-deamino-3'-aziridinyl-4'-methansulfonyl daunorubicin (Ia). That chemical compound, described in Anticancer Drug Design (1995), vol. 10, 641-653, is claimed in US-A-5,532,218 and is presently in the initial stage of clinical development. Compound Ia is able to circumvent the resistance to all major classes of cytotoxic compounds, indicating that the compound is a member of a new class of cytotoxic anti-tumor drugs, named alkycyclines. Its poor water solubility and low stability in aqueous media limit the therapeutic use of alkycycline.

EP-A-0 800 822 describes a method for preparing a closed vesicle by a rehydration of a liposome loading a pharmaceutically active substance using a rehydration solution; the method is characterized in that the rehydration is carried out at a low temperature and can provides a rehydrated stable vesicle without a leak of the loaded substance.

A number of attempts have been made to improve the stability of alkycyclines allowing a practical use of its high anti-tumor activity through the search of suitable compositions.

The alkycycline of formula Ia as defined above is quite water insoluble (less than 0.1 mg/ml). This makes it difficult to be formulated as a sterile, storage stable, dosage form. Additionally, the alkycycline is unstable:
after twelve months at 4°C the decomposition of the bulk product is about from 4 to 6%. This percentage of decomposition is absolutely not acceptable for a pharmaceutical product.

The attempts made to formulate a product with
a)cremophor and ethanol or
b)dimethylsulfoxide (DMSO)
completely failed. In the former case, the composition was only suitable for experimental purpose in the animals, but it was not stable for more than three days and caused allergic reactions.

From the latter composition, the dissolved alkycycline precipitates; moreover also this formulation is neither stable, nor suitable for intravenous (iv) administration, being a potential source of toxic side effects. Therefore, none of these formulations of alkycycline are pharmaceutically acceptable. There is a need for a stable pharmaceutical dosage form which may be conveniently administered to a cancer patient while keeping the stability of the alkycycline that is essential for the pharmacological activity.

It is an object of the present invention to provide a pharmaceutically acceptable buffered dosage form of an alkycycline of formula Ia as defined above having a pH value of from 4 to 10, more preferably of from 6 to 9. It is another object of the present invention to provide buffered lipid complexes of alkycycline. It is still another object of the invention to provide buffered lyophilized form of lipid complexes. The lyophilized form can be reconstituted with water, saline, or another electrolyte to give a colloidal dispersion for intravenous administration or can be formulated into a paste or filled into a soft gelatin or hard gelatin capsule for oral administration. The reconstituted composition of the invention has a pH value in the range of from 4 to 10, more preferably of from 6 to 9. Moreover, it appears that the lipid complex not only improves the stability, but also the pharmacokinetic properties and the distribution of the drug.

In accordance with the present invention, a lyophilized form of a phospholipid complex of alkycycline is prepared which can be reconstituted with pharmaceutically acceptable aqueous diluent such as water for injection and which, in comparison to other solutions, is less toxic, more stable and has an improved bio-distribution.

In accordance with the invention, the lipid complexes of alkycycline is prepared by a process comprising the steps of
a) preparing a solution of alkycycline, phospholipids and optionally cholesterol in an organic solvent,
b) adding an aqueous buffer,
c) removing the organic solvent by sparging an inert gas,
d) dissolving a pharmaceutically acceptable excipient (cryo-protectant in the aqueous phase of the dispersion,
e) reducing the size of the dispersion by conventional means and
f) lyophilizing the dispersion of the lipid complex to give a lyophilized form.

This lyophilized form gives a colloidal dispersion when reconstituted with a physiologically acceptable aqueous diluent. In accordance with a preferred embodiment of the present invention, the lyophilized form is prepared by a method comprising the steps of forming a concentrated solution of alkycycline in methylene chloride, dissolving the phospholipids dimyristoylphosphatidyl choline (DMPC) dimyristoylphosphatidyl glycerol (DMPG) and cholesterol in the same solvent, adding an aqueous solution with a phosphate buffer to form the lipid complex of alkycycline and provide a dispersion of the lipid complex in water as an aqueous phase, sparging and/or diafiltering the dispersion to remove the solvents, reducing the particle size of the dispersion of the lipid complex, adding an aqueous solution of mannitol as a pharmaceutically acceptable lyophilization excipient to the dispersion, and lyophilizing, wherein a lyophilizate is obtained which on reconstituting with water provides a colloidal dispersion of alkycycline lipid complex. In accordance with a further embodiment of the present invention, a lyophilized composition containing alkycycline is provided which comprises alkycycline, two phospholipids, optionally cholesterol and/or a buffer and a pharmaceutically acceptable lyophilization excipient. The term "lipid complex" is an art recognized term. Lipid complexes are characterized by a non-covalent bond between the lipid and the drug. The term "pharmaceutically acceptable aqueous diluent" as used herein refers to water for injection, saline, and other known aqueous vehicles. The term buffer as used herein refers to suitable buffers, for example phosphate buffer.

The term "lyophilization excipient" refers to a substance which is added to a solution prior to lyophilization to enhance characteristics such as the color, texture, strength, and volume of the cake.

Examples of lyophilization excipients are provided below In accordance with the invention a solution of alkycycline in an organic solvent is prepared. The most typical example of the solvent used to prepare this solution is methylene chloride.

However, other organic solvents such as chloroform, carbon tetrachloride, ethanol, iso-propyl alcohol and freon can be used. Useful solvents must form stable solutions with the alkycycline, e.g., the solvent must not interact with, destabilize, and/or deactivate the drug. In addition, the solubility of the alkycycline in the solvent must also be high enough that the alkycycline can be dissolved in amounts sufficiently high to form commercially useful quantities of the lipid complex and the solvent should be capable of being removed easily from an aqueous dispersion of the lipid complex as described hereinafter.

Preferably, a solution having a concentration of about 5 to 65 mg/ml, preferably about 20 to 60 mg/ml and most preferably 50 mg/ml alkycycline are used. The concentration may vary depending upon the nature of the solvent and temperature, but it is important to use a concentrated solution of the alkycycline in preparing the lipid/alkycycline complex. This minimizes the amount of solvent that must be removed later in the process, and also assists in forcing the alkycycline out of solution and into lipid/ alkycycline complex formation with the lipid with the addition of water. The organic solvent used to prepare the solution of the phospholipids should meet similar requirements to those outlined for the alkycycline solvent.

It must be compatible with the phospholipids and.not destabilize them or the alkycyclines. In addition, the lipids should be soluble enough in the solvent so as to be able to introduce enough of the lipid to form the complex yet minimize the amount of solvent that must be removed later. A solvent which can be readily removed from the dispersion of the lipid complex is most preferred. The solvent most typically used to prepare this solution is chloroform or methylene chloride. Typically the concentration of this phospholipid solution will range from about 10 to 250 mg/ml.

Phospholipids are amphipathic in nature, i.e., the molecules have a hydrophobic tail such as a long chain hydrocarbon, and a hydrophilic head. In an aqueous medium, such as water or saline, the tails align with each other, away from the aqueous molecules, while the heads face outward into the aqueous phase. It is this nature of the phospholipids that makes them very useful for formulation insoluble drugs like alkycycline.

The phospholipids used in the invention are selected such that their phase transition temperature is, preferably, about equal to or below the body temperature or 37°C and the complex releases the drug in the body. Representative examples of useful phospholipids include a combination of synthetic or semi- synthetic phospholipids chosen from DMPC, DMPG, dipalmitoylphosphatidylcholine (DPPC), dipalmitoylphosphatidylglycerol (DPPG), distearoylphosphatidylcholine(DSPC), distearoylphosphatidyl glycerol (DSPG), egg-phosphatidylcholine or egg-phosphatidylglycerol. Other examples of phospholipids can be found in the CRC Handbook of Lipid Bilayers by Marsh, M.., CRC Press (1990). When.DMPC and DMPG are used in a ratio of DMPC to DMPG of about 7:3 they mimic the human cell membrane. The phospholipid solution is added to the alkycycline solution such that the weight ratio of the alkycycline to phospholipid is about 1:80 to 1:3, preferably about 1:80 to 1:10, more preferably, about 1:60 to 1:10 and still more preferably about 1:45 to 1:25. It is important to add a buffer so that the pH value can be controlled.

In some applications, it has been found desirable to add cholesterol or its hemisuccinate derivatives to the phospholipid complex. The cholesterol is believed to cause the bilayers to pack more closely and thereby slow the release of the drug, and also to protect it from additional degradation. The cholesterol is added to the phospholipid solution. The cholesterol may be used in an amount of about 0.5 to 65 parts per 100 parts phospholipid.

Once the solvent solutions of the lipids and alkycycline have been mixed, an aqueous buffer is added rapidly and with stirring for several minutes the mixture. Addition of the aqueous buffer is believed to cause the alkycycline and the lipid to come out of their respective solvent solutions and complex with each other. The aqueous buffer is preferably added in an amount such that the alkycycline is present in an amount of about 0.05mg to 1.5mg per ml water. It is desirable to limit the amount of water to minimize the amount of water which must be removed during the lyophilization process. Higher amounts of water are undesirable because they increase the amount of water that must be removed during the subsequent lyophilization process. It is believed that the complexation may be complete in about 30 minutes. However, it is desirable to stir the dispersion for about one hour to ensure complex formation is complete. The lipid complex dispersion described above is treated to remove the solvents. Any of a variety of techniques can be used for this purpose. For example, it has been found that the methylene chloride or chloroform can be removed if the dispersion is sparged with a gas such as nitrogen.

Other techniques which can be used include centrifugation and diafiltration.

A pharmaceutically acceptable lyophilization excipient is dissolved in the aqueous phase of the dispersion. Mannitol is typically used as the excipient but other excipients which do not interact with the drug or the lipid complex may be used. Sodium or potassium phosphate, citric acid, tartaric acid, gelatin, and carbohydrates such as mannitol, lactose, dextrose, dextran, hetastarch, cyclodextrin, etc. are common examples of excipients which are also believed be useful herein. The excipients can be used alone or in combination to provide a cake of good quality which readily disperses in water upon reconstitution.

The excipients are typically added to the dispersion as solutions in water. Again, it is desirable to use concentrated solutions to minimize the amount of water for removal by lyophilization. The amount of the excipient is adjusted in a manner that is well known in the art to provide a cake which does not crack or shrink and is porous so that it readily dissolves and has a good appearance. Mannitol has been found to be useful. Mannitol is added to the dispersion as a solution having a concentration of about 5 to 200 mg/ml. Mannitol is added in an amount of about 1 to 100 parts by weight per 1 part alkycycline.

After removing the solvents and adding the excipient, the dispersion is passed through a homogenizer (e.g., a Tekmar rotor/stator homogenizer, Model T25, or a Microfluidics submerged jet homogenizer, Model M110Y). A dispersion having a particle size distribution ranging from about 10 to 5000nm and averaging about 400nm has been found to be satisfactory for lyophilization. The optimum particle size may vary depending on the mode of administration.

A typical lyophilization cycle useful in accordance with the present invention is provided below. The cycle may be varied depending upon the equipment and facilities available in a manner well known in the art. The homogenized formulation can be poured into vials of a 5 to 100ml nominal volume. The vials are placed into a lyophilization chamber at about 5°C. The vial size will usually be selected such that each vial contains a single dosage of the alkycycline. The temperature of the chamber is reduced to -30°C over a period of one hour after which the temperature is maintained at -5°C for about four hours.

The pressure in the lyophilization chamber is then reduced to 100-800 microns of pressure (13.3-107 pascals) for the remainder of the cycle. After reducing the pressure in the chamber, the temperature is ramped up to +20°C over a period of fifteen hours and the product is held at 25°C for five hours. The temperature then is ramped up to +40°C over a period of 20 minutes and held at 40°C for two hours. The lyophilized product preferably has a final moisture content of less than about 5% and typically about 1 to 2 %.

The pharmaceutical form of the present invention can be administered to a patient intravenously or formulated for oral use in the treatment of cancer and other diseases.

For intravenous administration, the lyophilizate can be reconstituted using aqueous vehicles such as water, saline or another electrolyte. The lyophilized product with the addition of water provides a colloidal dispersion of the lipid complex in an aqueous solution of the excipient. Neither the alkycycline nor the lipids are soluble in water. A colloidal dispersion consists of at least two discrete phases. The first is a dispersed or internal phase. The second is a continuous or external phase. Systems in the colloidal state contain one or more substances that have at least one dimension in the range of 10-100nm to a few microns. See pp. 272-4 in Chapter 19, Disperse Systems, Remington's Pharmaceutical Sciences, 18th Edition, 1990, Mack Publishing Company, Easton, PA 18042. In the colloidal dispersions of the present invention, the dispersed or internal phase comprises majority of particles of alkycycline lipid complex having a particle size in the range of 10nm to 2000nm. In selecting the aqueous vehicle, it is recommended to use one having a specific gravity about equal to the lipid complex (est.1.09g/ml) to minimize the tendency for the dispersion to settle. The lyophilizate of the lipid complex can be reconstituted with water, saline, or another pharmaceutically acceptable aqueous diluent for intravenous administration. Upon reconstitution, a dispersion is obtained which is suitable for injection.

The lyophilizate can also be administered orally as an aqueous dispersion or as a paste. For oral administration, the lyophilizate can be reconstituted to form an oral dispersion or formulated into a paste. Alternatively, the lyophilizate can be filled into a soft gelatin capsule for oral administration.

The object of the present invention is useful in a method of treatment of cancer by administering the dosage form of alkycycline of from about 1 to about 25 mg/m² of body surface area. More preferably, the course therapy employed is from about 2 to about 18 mg/m² of body surface area once a week or once every three weeks. When given weekly, the drug is administered at a dose of 2 to 6 mg/m² for four weeks followed from two week interruption. The drug is preferably administered as a bolus administration or as continuous infusion over from 10 minutes to 12 hours, preferably from 10 minutes to 4 hours using programmable continuous infusion ambulatory pump or iv infusion bags.

The present anti-tumor agent exerts a strong anti-tumor activity on human malignant tumors, for example, on solid tumors such as gastrointestinal tumors, like colorectal cancer, gastro-esophageal cancer, cancer of liver and biliary tract, pancreatic cancer; prostatic cancer, testicular cancer, lung cancer, breast cancer, malignant melanoma, brain tumors (such as glioma and astrocytomas), ovarian cancer, uterine cancer including cervical cancer, cancer of the head and neck, bladder cancer, Kaposi sarcoma, including AIDS-related Kaposi sarcoma, sarcomas and osteosarcoma, renal carcinoma; and hematopoietic malignant tumors such as leukemia and lymphoma, including AIDS-related lymphomas.

While it is contemplated herein that the lipid/alkycycline complex will be lyophilized to enhance its stability, it will be appreciated that the lipid/alkycycline complex is pharmaceutically active and can be formulated into a dosage form for oral and intravenous administration without lyophilization. Formulation aids such as antibacterials and antioxidants can be used to enhance the stability of the complex.

The invention will now be described in more detail with reference to the following non-limiting examples.

### Example 1

5 g of 4-demethoxy-3'-deamino-3'-aziridinyl-4'-methansulfonyl daunorubicin were dissolved in about 100ml of methylene chloride. To this solution were added 95 g of DMPC, 30g of DMPG, 40 g of cholesterol dissolved in about 1.7 l of methylene chloride, and the resultant mixture was stirred till complete dissolution. To the above solution were added about 4.61 of phosphate buffer at a pH of 8.5. The two-phase system was stirred using a laboratory stirrer and then sparged with nitrogen till the level of methylene chloride was less than 1%. To this solution was added a solution of mannitol (250 g in 51 of WFI). The suspension was then homogenized using a Microfluidics Model M110Y homogenizer, filled in 50 cc, clean, moulded vials and freeze dried.

The freeze dried product was placed on long term and accelerated stability. After 18 months of storage at -20°C and + 5°C, the product was seen to have over 90% of its initial potency.

### Example 2

Two phase I and pharmacological trials investigate the I.V. administration of alkycycline composition prepared by reconstituting the lyophilizate of the lipid complex obtained according to example 1 once every 3 weeks in patients with solid tumors, including lung, colorectal, gastro-oesophageal, pancreatic, renal, head and neck, ovarian cancer, sarcoma and melanoma.

Starting dose, 1 mg/m², was escalated with an initial accelerated phase (1 patient / dose level) followed by conventional dose escalation in 3-6 patient cohorts.

Overall, 53 patients were entered, 40 patients (median age 56.5 years; median ECOG PS 1) and 105 cycles were evaluated for non-hematological toxicity, 52 patients and 118 cycles were evaluated for hematological toxicity. Dose levels studied were 1, 2, 4, 6, 9, 12, 14 and 16 mg/m². Non-hematological toxicities mainly consisted of nausea and vomiting and were usually mild to moderate. At different dose levels (2, 6, 9 and 12 mg/m² [1 patient each] and 14 mg/m² [2 patients]) a histamine release syndrome was observed during or shortly after drug administration, which recovered either spontaneously or with anti-histamine standard therapy. Thrombocytopenia was the main hematologic toxicity and occurred in 27/52 patients during the first cycle, starting from 6 mg/m² and was dose limiting (defined as Platelets 50 - 25 x 10⁹/L for 5 days or < 25.0 x 10⁹/L) at 6 (1/6 patients), 9 (1/10), 12 (1/16) and 14 mg/m² (3/14).

Observations at 16 mg/m² are ongoing, and further dose increases are foreseen.

Two patients with NSCLC, who had failed previous standard therapy, achieved disease stabilization for 30 and 19 weeks, respectively (one patient still on treatment).

One patient with renal cancer, who had failed previous standard therapy, had stable disease for 18 weeks.

Details about three cases are reported here below:
Patient 001010 (study 98OALK002) was a 65 year-old man with a diagnosis of NSCLC initially treated with surgery (bilobectomy of the right lung). Four years after surgery the patient relapsed and received 3 cycles of chemotherapy including cisplatin and velbe, which resulted to be ineffective, in fact the best tumor response to this regimen was progression of the disease. Another chemotherapy treatment with an analog of taxol (which is known to be an effective agent in NSCLC) was then started. During this treatment the disease was stabilized for 14 weeks. Ultimately tumor progression occurred again in the chest and the patient entered in the present study. Eight cycles of the study drug at 9 mg/m² were administered, producing a stabilization of the disease lasting significantly longer as compared to the previous therapy (30 weeks). The patient was finally taken off treatment for tumor progression and was still alive as of October 15, 1999.
Patient 001014 (study 98OALK001) was a 69 year-old man with NSCLC, who underwent a lobectomy of the upper lobe of right lung followed by a right rest pneumectomy six years later. After three years the patient progressed and received 2 cycles of cisplatin plus vepesid. Despite the therapy, disease progression occurred in the chest within one month and the patient entered in our study. This rapid tumor progression was halted by the study drug administered at 12 mg/m² for six cycles, and the tumor remained stable for 19 weeks. The patient went off study at the end of the sixth treatment cycle.
Patient 001015 (study 98OALK002) was a 52 year-old man with renal carcinoma, who underwent left nefrectomy plus splenectomy, followed by velbe plus interferon alfa (21 cycles). The disease was stabilized for 1.5 year, after which both local (left kidney) and distant (lung and liver) progression occurred and the patient entered in our study. Six cycles of study drug at 12 mg/m² have been administered so far (overall, patient has been on treatment for 18 weeks) with disease stabilization. The patient was still on treatment as of October 4, 1999.

## Claims

1. A buffered lyophilized form of a lipid complex comprising:
- an alkycycline of formula Ia 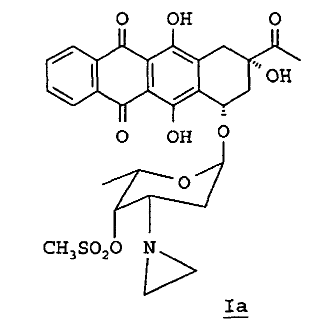
- two phospholipids,
- a buffer and
- a pharmaceutically acceptable lyophilization excipient.

2. A buffered lyophilized form according to claim 1 which forms a colloidal dispersion when reconstituted with a physiologically acceptable aqueous diluent.

3. A buffered lyophilized form according to claim 1 wherein the phospholipids are selected from dimyristoylphosphatidyl choline, dimyristoylphosphatidyl glycerol, dipalmitoyliphosphatidyl choline, dipalmitoylphophatidyl glycerol, distearoylphosphatidyl choline, distearoylphosphatidyl glycerol, egg-phosphatidylcholine and egg-phosphatidyl glycerol.

4. A buffered lyophilized form according to claim 1 wherein said phospholipid is a mixture of dimyristoylphosphatidyl choline and dimyristoylphosphatidyl glycerol.

5. A buffered lyophilized form according to claim 4 wherein the dimyristoylphosphatidyl choline is present in a weight ratio to the dimyristoylphosphatidyl glycerol of about 7:3.

6. A buffered lyophilized form according to claim 1 wherein the ratio of the alkycycline of formula Ia as defined in claim 1 to the phospholipid is from 1:80 to 1:3.

7. A buffered lyophilized form according to claim 1 wherein the lyophilisation excipient is mannitol.

8. A buffered lyophilized form according to claim 1 which further comprises cholesterol.

9. A pharmaceutically acceptable buffered dosage form of alkycycline having a pH value of from 4 to 10, formed by reconstituting with a physiologically acceptable aqueous diluent.a buffered lyophilized form according to claim 1.

10. A bufferd lipid complex comprising:
- an alkycycline of formula Ia 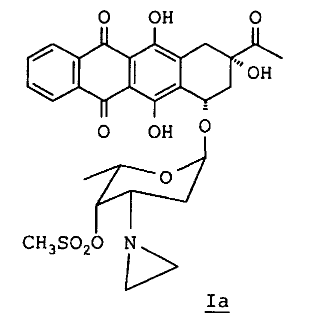
- two phospholipids,
- a buffer and optionally
- a formulation aid, formulated into a dosage form for oral or intravenous administration.

## Patentansprüche

1. Gepufferte lyophilisierte Form eines Lipidkomplexes, der
- ein Alkycyclin mit der Formel Ia 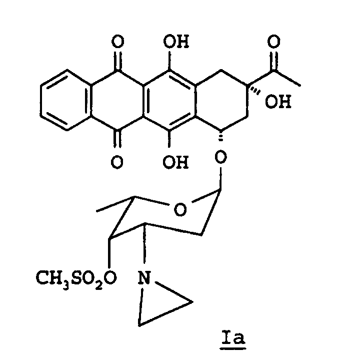
- zwei Phospholipide,
- einen Puffer und
- ein pharmazeutisch annehmbares Lyophilisierungshilfsmittel umfasst.

2. Gepufferte lyophilisierte Form nach Anspruch 1, die eine kolloidale Dispersion bildet, wenn sie mit einem physiologisch annehmbaren wässrigen Verdünnungsmittel rekonstituiert wird.

3. Gepufferte lyophilisierte Form nach Anspruch 1, bei dem die Phospholipide ausgewählt sind aus Dimyristoylphosphatidylcholin, Dimyristoylphosphatidylglycerin, Dipalmitoylphosphatidylcholin, Dipalmitoylphosphatidylglycerin, Distearoylphosphatidylcholin, Distearoylphosphatidylglycerin, Phosphatidylcholin aus Ei und Phosphadidylglycerin aus Ei.

4. Gepufferte lyophilisierte Form nach Anspruch 1, bei dem das Phospholipid eine Mischung aus Dimyristoylphosphatidylcholin und Dimyristoylphosphatidylglycerin ist.

5. Gepufferte lyophilisierte Form nach Anspruch 4, bei der das Dimyristoylphosphatidylcholin in einem Gewichtsverhältnis zu dem Dimyristoylphosphatidylglycerin von etwa 7:3 vorliegt.

6. Gepufferte lyophilisierte Form nach Anspruch 1, bei der das Verhältnis des Alkycyclins der Formel Ia wie in Anspruch 1 definiert zu dem Phospholipid 1:80 bis 1:3 beträgt.

7. Gepufferte lyophilisierte Form nach Anspruch 1, bei der das Lyophilisierungshilfsmittel Mannitol ist.

8. Gepufferte lyophilisierte Form nach Anspruch 1, die des Weiteren Cholesterin umfasst.

9. Pharmazeutisch annehmbare gepufferte Dosierform von Alkycyclin mit einem pH-Wert von 4 bis 10, die durch Rekonstituieren einer gepufferte lyophilisierten Form nach Anspruch 1 mit einem physiologisch annehmbaren wässrigen Verdünnungsmittel gebildet wird.

10. Gepufferter Lipidkomplex, der
- ein Alkycyclin mit der Formel Ia 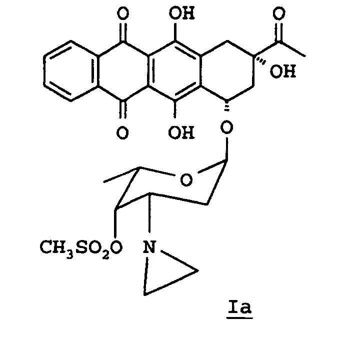
- zwei Phospholipide,
- einen Puffer und
- ein Formulierungshilfsmittel umfasst,
formuliert zu einer Dosierform zur oralen oder intravenösen Verabreichung.

## Revendications

1. Forme tamponnée et lyophilisée d'un complexe lipidique comprenant :
- une alkycycline de la formule Ia 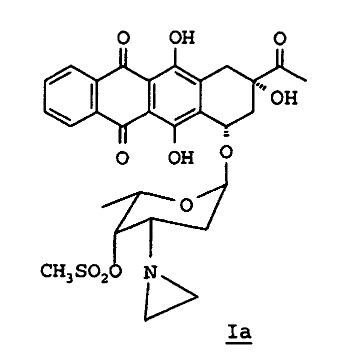
- deux phospholipides,
- un tampon, et
- un excipient de lyophilisation acceptable sur le plan pharmaceutique.

2. Forme tamponnée et lyophilisée selon la revendication 1, qui forme une dispersion colloidale lorsqu'elle est reconstituée avec un diluant aqueux acceptable sur le plan physiologique.

3. Forme tamponnée et lyophilisée selon la revendication 1, dans laquelle les phospholipides sont choisis parmi la dimyristoylphosphatidylcholine, le dimyristoylphosphatidyl-glycérol, la dipalmitoylphosphatidylcholine, le dipalmitoylphosphatidylglycérol, la distéaroylphosphatidyl-choline, le distéaroylphosphatidylglycérol, la phosphatidylcholine d'oeufs, et le phosphatidylglycérol d'oeufs.

4. Forme tamponnée et lyophilisée selon la revendication 1, dans laquelle ledit phospholipide est un mélange de dimyristoylphosphatidylcholine et de dimyristoylphosphatidyl-glycérol.

5. Forme tamponnée et lyophilisée selon la revendication 4, dans laquelle la dimyristoylphosphatidylcholine est présente dans un rapport pondéral par rapport au dimyristoylphosphatidylglycérol d'environ 7/3.

6. Forme saline et lyophilisée selon la revendication 1, dans laquelle le rapport de l'alkycycline de la formule Ia telle que définie dans la revendication 1 par rapport au phospholipide est de 1/80 à 1/3.

7. Forme saline et lyophilisée selon la revendication 1, dans laquelle l'excipient de lyophilisation est le mannitol.

8. Forme saline et lyophilisée selon la revendication 1, qui comprend en outre le cholestérol.

9. Forme posologique tamponnée acceptable sur le plan pharmaceutique d'alkycycline ayant une valeur de pH comprise entre 4 et 10, formée par la reconstitution avec un diluant aqueux acceptable sur le plan physiologique d'une forme tamponnée et lyophilisée selon la revendication 1.

10. Complexe lipidique tamponné comprenant :
- une alkycycline de la formule Ia 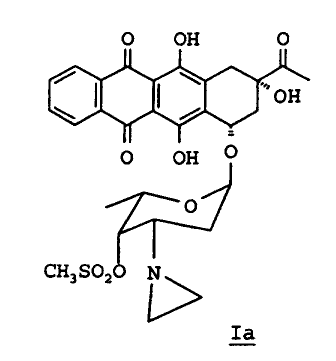
- deux phospholipides,
- un tampon, et facultativement,
- un adjuvant de formulation, formulé en une forme posologique destinée à une administration orale ou intraveineuse.
